# EUROPEAN PATENT SPECIFICATION

(11) **EP 1 757 297 B1**
(45) Date of publication and mention of the grant of the patent: **13.11.2013**
(21) Application number: 05743564.6
(22) Date of filing: 26.05.2005
(51) Int. Cl.: A61K 31/7004, A23K 1/16, A23L 1/30, A61P 9/10, A61P 43/00, C07H 3/02

(54) **COMPOSITION FOR treating ARTERIOSCLEROSIS IN DIABETIC PATIENTS**
ZUSAMMENSETZUNG ZUR Behandlung von ARTERIOSKLEROSE IN DIABETISCHEN PATIENTEN
COMPOSITION pour le traitement DE L'ARTÉRIOSCLÉROSE ET PROCÉDÉ D'INHIBITION CHEZ LES PATIENTS DIABÉTIQUES

(30) Priority: 26.05.2004 JP 2004155672
(43) Date of publication of application: 28.02.2007
(73) Proprietor: National University Corporation Kagawa University, Takamatsu, Kagawa 760-8521 (JP); Matsutani Chemical Industry Co., Ltd., Itami-shi Hyogo 664-8508 (JP)
(72) Inventor: TOKUDA, Masaaki; c/o Faculty of Medicine, Kagawa U, Mikicho, Kitagunawa;7610793 (JP); MURAO, Kouji; c/o Faculty of Medicine, Kagawa Univ, Kagawa ;7610793 (JP); ISHIDA, Toshihiko; c/o Faculty of Medicine, Kagawa, kagawa;7610793 (JP); IZUMORI, Ken; c/o Rare Sugar Research Center,, Kagawa, 761- 0795 (JP)
(74) Representative: TBK
(86) International application number: PCT/JP2005/009690
(87) International publication number: WO 2005/115409

(56) References cited:
- EP-A1- 1 538 200
- WO-A-2005/020721
- WO-A1-03/097820
- WO-A1-03/097820
- MATSUO T ET AL: "Less Body Fat Accumulation with D-Psicose Diet versus D-Fructose Diet" JOURNAL OF CLINICAL BIOCHEMISTRY AND NUTRITION, TOKYO, JP, vol. 30, 2001, pages 55-65, XP003003027 ISSN: 0912-0009

## Description

### Technical Field

The present invention relates to a technique of inhibiting the induction of the expression of a chemokine by a cytokine related to aggravation of arteriosclerosis and promoting the expression of a receptor involved in the improvement of arteriosclerosis without affecting the expression of a scavenger receptor CD36 which is an arteriosclerosis inducer by a rare sugar.

### Background Art

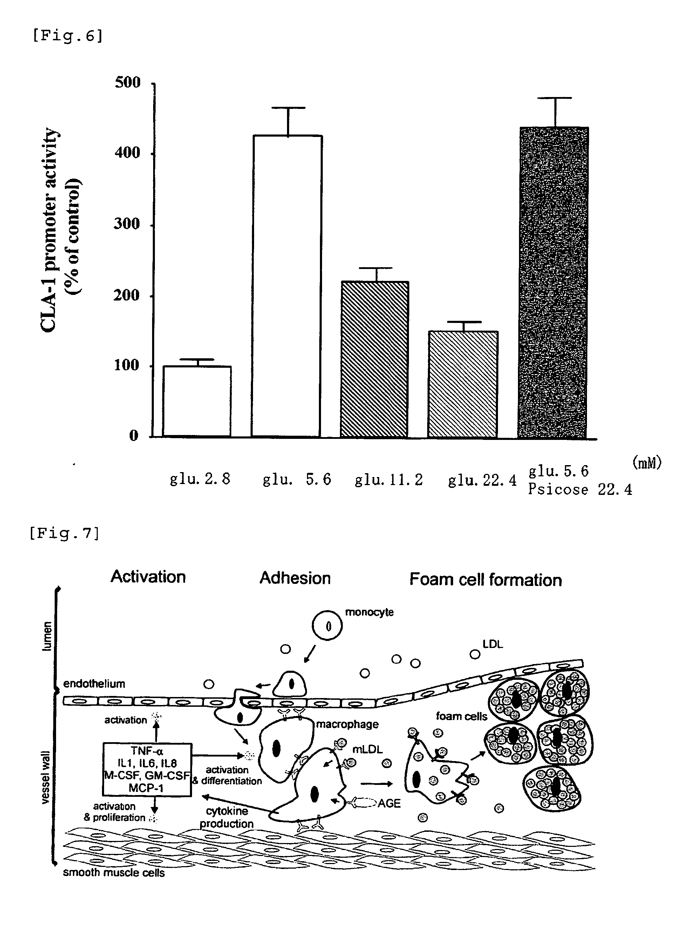

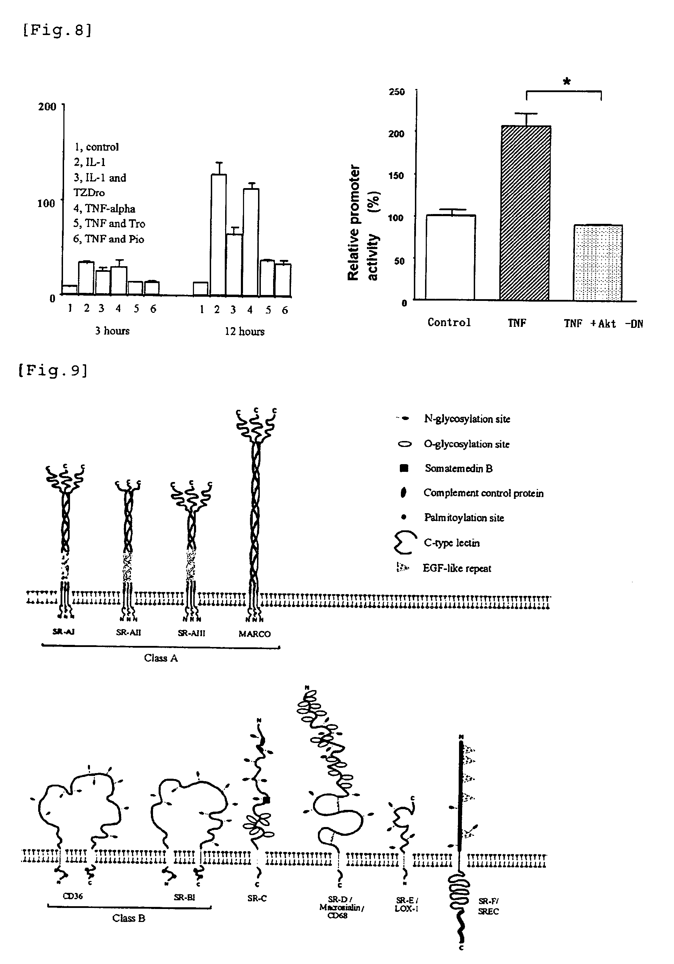

Deaths attributable to arteriosclerosis are the top of the causes of death. As the risk factors of arteriosclerosis, diabetes, hyperlipemia, hypertension and the like have been pointed out. According to the recent reports, analyses at the molecular level have progressed on the mechanisms of these risk factors and the onset of arteriosclerosis. The trigger of the onset of arteriosclerosis is the migration of monocytes to the vascular wall and cholesterol accumulation by scavenger receptors expressed in macrophages,resulting the form cell formation (Fig. 7, Non-Patent Documents: 13 and 18).
It has been already reported that the secretion of a chemokine MCP-1, which is a factor secreted from vascular endothelial cells in the arteriosclerotic lesion and related to the migration of monocytes, is inhibited by D-psicose and the promotion of MCP-1 secretion by a cytokine is inhibited, as a result the antiatherogenic action of D-psicose has been reported (Patent Document 1, Non-Patent Documents: 6, 9-11 and 17).
On the other hand, a scavenger receptor, which is a receptor for oxidized LDL, the most important atherogenetic lipid , is expressed by macrophages in the arteriosclerotic lesion and plays the leading role in the formation of arteriosclerotic lesion. (Fig. 9, Patent Documents: 5, 13 and 19).

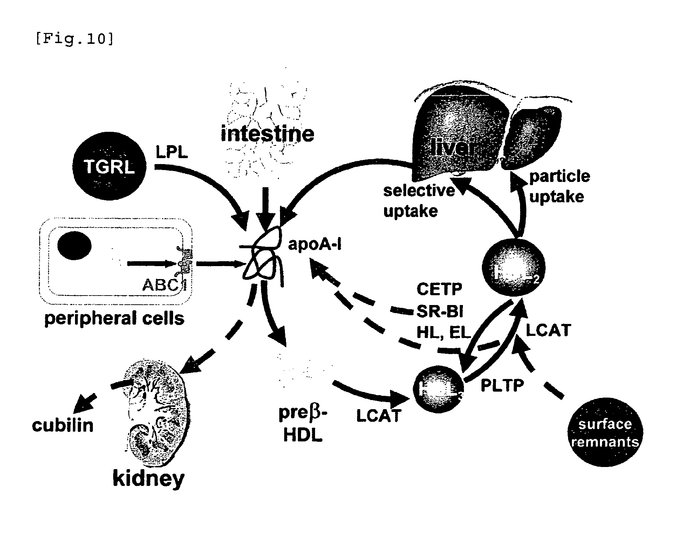

Incidentally, the main symptom of diabetes is a vascular disorder and the cause of death is due to arteriosclerosis. The details of the mechanism of the onset of arteriosclerosis by diabetes are not clear. Recently, it has been reported that the expression of a scavenger receptor CD36 is induced in hyperglycemic conditions, which has attracted attention (Non-Patent Documents: 1 and 20).
On the other hand, the reverse cholesterol transport system mediated by high density lipoprotein (HDL) is known as an antiatherogenic action in vivo (Fig. 10, Non-Patent Documents: 14 and 16).
We have identified a human gene, CLA-1 as a receptor for HDL (Non-Patent Documents: 2, 3, 8, 12 and 15).
From the study of gene introduction, it is known that CLA-1 plays an important role on the reverse cholesterol transport system via the selective cholesterol ester uptake from HDL in the liver (Non-Patent Documents: 4, 7, 14 and 15) .

Patent Document 1: WO 03/097820
Non-Patent Document 1: Yu X, Murao K, Sayo Y, Imachi H, Cao WM, Ohtsuka S, Niimi M, Tokumitsu H, Inuzuka H, Wong NC, Kobayashi R, Ishida T "The Role of Calcium/Calmodulin-Dependent Protein Kinase Cascade in Glucose Upregulation of Insulin Gene Expression" Diabetes 53, 1475-1481, 2004.
Non-Patent Document 2: Cao WM, Murao K, Imachi H, Yu X, Abe H, Yamauchi A, Wong NC, Ishida T "A Mutant HDL Receptor Inhibits Proliferation of Human Breast Cancer Cells." Cancer Res 64, 1515-1521, 2004
Non-Patent Document 3: Cao WM, Murao K, Imachi H, Hiramine C, Yu X, Wong NC, Takahara J, Ishida T "Phosphatidylserine Receptor Cooperates with High Density Lipoprotein Receptor on Recognition of Apoptotic Cells by Thymic Nurse Cells." J Mol Endocrinol 32, 497-505, 2004
Non-Patent Document 4: Imachi H, Murao K, Cao WM, Tada S, Taminato T, Wong NC, Takahara J, Ishida T "Expression of human scavenger reveptor B1 on and in human platelets" Arterioscler. Thromb. Vasc. Biol. 23: 898-904, 2003
Non-Patent Document 5: Cao WM, Murao K, Imachi H, Nakano T, Kodama T, Wong NC, Takahara J, Ishida T "PI3 kinase-Akt/ PKB pathway mediates Gas6 induction of SRA in vascular smooth muscle cell" Arterioscler Thromb Vasc Biol 21: 1592-1597, 2001 Non-Patent Document 6: Momoi A, Murao K, Imachi H, Ishida T, Cao WM, Sato M, Takahara J "Inhibition of monocyte chemoattractant protein-1 (MCP-1) expression in cytokine-treated human lung epithelial cells by thiazolidinedione" CHEST 120: 1293-1300, 2001
Non-Patent Document 7: Imachi H, Murao K, Cao WM, Ohyama T, Sato M, Sasaguri Y, Ishida T, Takahara J "Expression of HDL receptor, CLA-1 in human smooth-muscle cells and effect of interferon-gamma on its regulation." Horm Metab Res 33: 389-393; 2001
Non-Patent Document 8: Imachi H, Murao K, Hiramine C, Sayo Y, Sato M, Hosokawa H, Ishida T, Kodama T, Quehenberger O, Steinberg D, Takahara J "Human scavenger receptor B1 is involved in recognition of apoptotic thymocytes by nurse cells." Lab Invest 80: 263-70; 2000
Non-Patent Document 9: Murao K, Ohyama T, Imachi H, Ishida T, Cao WM, Sato M, Wong NC, Takahara J "TNF-alpha stimulation of MCP-1 expression is mediated by the Akt/PKB signal transduction pathway in vascular endothelial cells." Biochem Biophys Res Commun 276: 791-796; 2000
Non-Patent Document 10: Momoi A, Murao K, Imachi H, Sayo Y, Nakamura H, Hosokawa H, Sato M, Fujita J, Okada H, Ishida T, Takahara J "Thiazolidinedione inhibits production of RANTES in a cytokine-treated human lung epithelial cell line." FEBS Lett 452: 301-304; 1999
Non-Patent Document 11: Murao K, Imachi H, Momoi A, Sayo Y, Hosokawa H, Sato M, Ishida T, Takahara J "Thiazolidinedione inhibits the production of monocyte chemoattractant protein-1 in cytokine-treated human vascular endothelial cells." FEBS Lett 454: 27-30; 1999
Non-Patent Document 12: Imachi H, Murao K, Sato M, Hosokawa H, Ishida T, Takahara J "CD 36 LIMPII analogous-1, a human homolog of the rodent scavenger receptor B1, provides the cholesterol ester for steroidogenesis in adrenocortical cells." Metabolism 48: 627-30; 1999
Non-Patent Document 13: Murao K, Imachi H, Sayo Y, Hosokawa H, Sato M, Ishida T, Nakano T, Kodama T, Sasaguri Y, Takahara J "A product of growth arrest-specific gene 6 modulates scavenger receptor expression in human vascular smooth muscle cells." FEBS Lett 459: 363-6; 1999
Non-Patent Document 14: Murao K, Wada Y, Nakamura T, Taylor AH, Mooradian AD, Wong NC "Effects of glucose and insulin on rat apolipoprotein A-I gene expression." J. Biol. Chem. 273: 18959-65, 1998
Non-Patent Document 15: Murao K, Teraptca V, Green SR, Kondratenko K, Steinberg D, Quenquenberger O. "Characterization of CLA-1, Human Homologue of Rodent Scavenger Receptor B1 as Receptor for HDL and apoptotic thymocyte." J Biol Chem 272, 17551-17557, 1997
Non-Patent Document 16: Murao K, Bassyouni H, Taylor AH, Wanke IE, Wong NCW "Hepatocyte Nuclear Factor 4 Inhibits Activity of Site A from the Rat Apolipoprotein A1 gene." Biochemistry. 36, 301-306, 1997
Non-Patent Document 17: Tangirara R, Murao K, Quenquenberger O. "Regulation of the monocyte chemotactic protein-1 receptor expression by cytokines." J Biol Chem 272, 8050-8056, 1997
Non-Patent Document 18: Tall AR "Plasma high density lipoproteins. Metabolism and relationship to atherogenesis. J Clin Invest 86: 379-384; 1990
Non-Patent Document 19: Steinberg D. "Low density lipoprotein oxidation and its pathobiological significance." J Biol Chem 272: 20963-20966; 1997
Non-Patent Document 20: Griffin E, Re A, Hamel N, Fu C, Bush H, McCaffrey T, Asch AS. "A link between diabetes and atherosclerosis: Glucose regulates expression of CD36 at the level of translation." Nat Med 7: 840-846, 2001
WO-A-2005/020721 is directed to beverage compositions, which may comprise D-psicose.
MATSUO T ET AL: "Less Body Fat Accumulation with D-Psicose Diet versus D-Fructose Diet" JOURNAL OF CLINICAL BIOCHEMISTRY AND NUTRITION, TOKYO, JP, vol. 30, 2001, pages 55-65, describes dietary food comprising D-psicose.
WO-A-03 0 978 820 describes a method of utilizing physiological activity of rare saccharides and composition containing such rare saccharides to modify the function of cells.

### Disclosure of the invention

### Problems that the Invention is to Solve

Monocyte chemoattractant protein-1 (MCP-1) is a chemokine belonging to the C-C family, and is secreted from various cells including vascular endothelial cells, and has a chemotactic activity for monocytes.
In the arteriosclerotic lesion, MCP-1 is released from damaged vascular endothelial cells, and induces a migration of monocytes to the vascular wall and differentiation thereof into macrophages, and contributes the formation of early lesion of arteriosclerosis.
As stimulation of MCP-1 secretion, inflammatory cytokines, TNF-α and IL-1β are known (Fig. 8), and as an intracellular signal transduction system, PI3-K/PKB(AKT) pathway is involved in the stimulation of MCP-1 by cytokines (Fig. 8), and on the contrary, thiazolidinedione (TZD) is a high-affinity ligand for the PPAR-γ acts on an inhibitory effect on cytokine-induced MCP-1 secretion.
According to the previous reports, a high concentration of glucose stimulates the secretion of MCP-1, and also clinically, it has been pointed out that the level of MCP-1 is high in patients with diabetes.
Therefore, a compound that inhibits the secretion of MCP-1 from vascular endothelial cells and a compound that inhibits the secretion of MCP-1 by an inflammatory cytokine IL-1β will be an excellent preventive and/or therapeutic agent for arteriosclerosis in patients with diabetes, and the development of a technique associated with them have been demanded.

Accordingly, the present inventors examined an effect of a rare sugar, D-psicose, on a group of scavenger receptors and also HDL receptor, and aimed at finding effectiveness of the rare sugar for arteriosclerosis and a possibility of the clinical application thereof. Thus, the present invention has its object to experimentally support an action of inhibiting the induction of the expression of a chemokine by a cytokine related to aggravation of arteriosclerosis and promoting the expression of a receptor involved in the improvement of arteriosclerosis without affecting the expression of a scavenger receptor CD36 which is an arteriosclerosis inducer by D-psicose, and to provide a composition for use in treating arteriosclerosis in patients with diabetes related to the action

### Means for Solving the Problems

A gist of the present invention is a composition for use in treating arteriosclerosis in patients with diabetes according to any of the following (1) to (5).
(1) A composition for use in treating arteriosclerosis in patients with diabetes, characterized by comprising a rare sugar, D-psicose as an active ingredient for an antiatherogenic action.
(2) The composition for inhibiting the onset of arteriosclerosis in patients with diabetes according to the above (1), wherein the antiatherogenic action includes an action of inhibiting or reducing the expression of a chemokine or a cytokine related to arteriosclerosis in vivo and an action of promoting the expression of a receptor involved in the improvement of arteriosclerosis without affecting the expression of an arteriosclerosis inducer.
(3) The composition for inhibiting the onset of arteriosclerosis according to the above (2), wherein the action of promoting the expression is an action of inhibiting the induction of the expression of a chemokine by a cytokine related to arteriosclerosis in vivo and promoting the expression of a receptor involved in the improvement of arteriosclerosis without affecting the expression of a scavenger receptor CD36 which is an arteriosclerosis inducer by D-psicose.
(4) The composition for inhibiting the onset of arteriosclerosis according to the above (1), (2) or (3), wherein D-psicose is used in the form of a composition blended with D-psicose and/or a mixture thereof.
(5) The composition for inhibiting the onset of arteriosclerosis according to the above (4), wherein the composition is in the form of a product selected from the group consisting of sweeteners, seasonings, food additives, food materials, foods and drinks, health foods and drinks, drugs, and feeds blended with D-psicose and/or a mixture thereof as the active ingredient.

### Advantage of the Invention

According to the present invention, a composition for use in treating arteriosclerosis in patients with diabetes using D-psicose (a sweetener, a seasoning, a food additive, a food material, a food or drink, a health food or drink, a drug, a quasi drug and a feed) is provided.

### Best Mode for Carrying Out the Invention

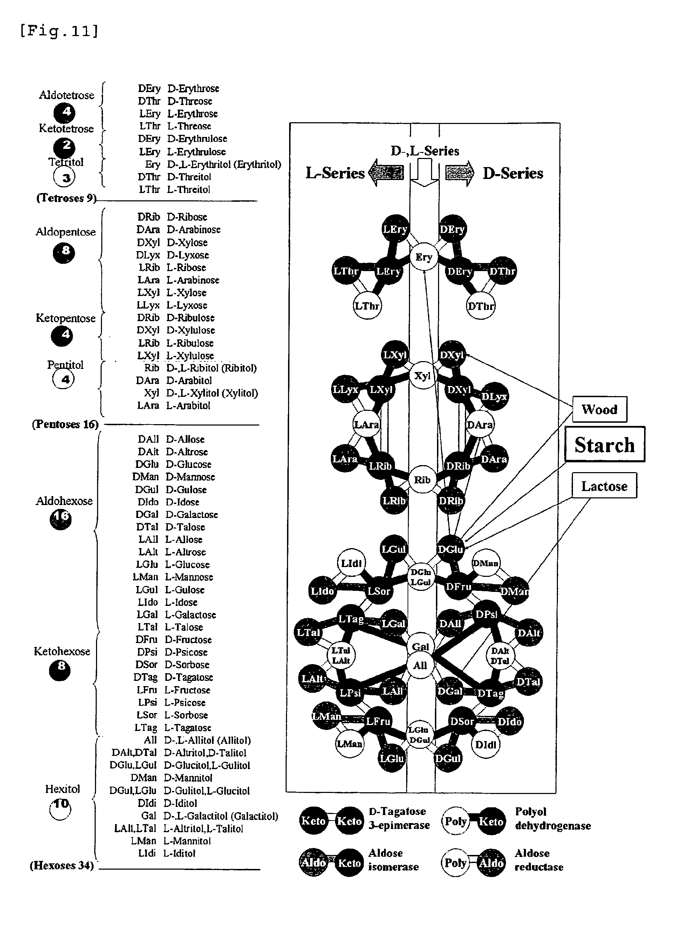

The present invention relates to a technique utilizing an action of inhibiting the induction of the expression of a chemokine by a cytokine related to arteriosclerosis in vivo (for example, inhibition of the secretion of MCP-1) and promoting the expression of a receptor (for example, an HDL receptor CLA-1) involved in the improvement of arteriosclerosis without affecting the expression of a scavenger receptor CD36 which is an arteriosclerosis inducer (for example, induction of arteriosclerosis by increasing the expression thereof due to high blood sugar levels) by D-psicose. Accordingly, the rare sugar D-psicose used in the present invention will be described.
The "rare sugar" can be defined as a monosaccharide that exists only in a small amount in nature. There are 7 types of monosaccharides that exist in a large amount in nature, which are D-glucose, D-fructose, D-galactose, D-mannose, D-ribose, D-xylose and L-arabinose, and the other monosaccharides exist in a small amount in nature and can be classified into a rare sugar. Further, a sugar alcohol can be produced by reducing a monosaccharide, and D-sorbitol and D-mannitol exist in a relatively large amount in nature, however, the other sugar alcohols exist in a small amount, therefore, these can also be defined as a rare sugar in accordance with the present invention. It has been difficult to obtain such a rare sugar so far, however, a process for producing a rare sugar from a monosaccharide that exists in a large amount in nature is being developed, and it can be produced by utilizing the technique.
Hereinafter, description based on Izumoring (registered trademark, hereinafter omitted) proposed in order to more easily understand a correlation of these monosaccharides will be added (see WO 03/097820).
A linkage diagram in which all the monosaccharides having 4 to 6 carbon atoms are linked together based on their production processes and molecular structures (D-form and L-form) shown in Fig. 11 is the overall diagram of Izumoring. That is, what one can understand from Fig. 11 is that monosaccharides having 4, 5 and 6 carbon atoms are all linked together. In the overall diagram, the members in Izumoring of C6 are linked together, the members in Izumoring of C5 are linked together, the members in Izumoring of C4 are linked together, and Izumorings of C4, C5 and C6 are all linked together. This concept is important. In order to reduce the number of carbon atoms, a fermentation method is mainly used. It is also characterized by being a big linkage diagram in which all the monosaccharides having different number of carbon atoms are linked together.

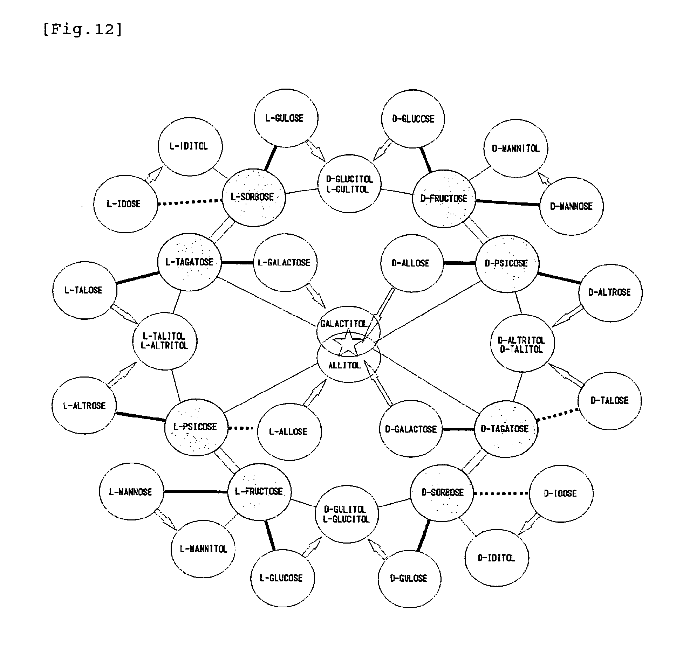

In Izumoring of the monosaccharides having 6 carbon atoms (hexoses), as shown in the lower portion of Fig. 11 and Fig. 12, there are a total of 34 types of monosaccharides having 6 carbon atoms (hexoses) including 16 types of aldoses, 8 types of ketoses and 10 types of sugar alcohols. It is known by the studies including the studies performed by the present inventors that these sugars can be converted by an oxidoreductase reaction, an aldose isomerization reaction or an aldose reductase reaction.
However, the upper group, the middle group and the lower group were not linked together by an enzymatic reaction in the conventional studies. In other words, although D-glucose (glucose) or D-fructose belonging to the upper group is a sugar that exists in a large amount in nature and is inexpensive, a rare sugar could not be synthesized from this sugar. However, in the process of the study performed by the present inventors, an enzyme that links these was found. D-sorbose which was completely unexpectedly found in a culture solution of a bacterium having an enzyme that synthesizes D-tagatose from galactitol, which was the beginning of the finding of the enzyme. From the results of examining the cause, it was found that this bacterium produces an enzyme called D-tagatose-3-epimerase (DTE).
As shown in the lower portion of Fig. 11 and Fig. 12, it is understood that this DTE is an enzyme that connects between D-tagatose and D-sorbose which was disconnected so far. Further surprisingly, it was found that this DTE is an enzyme that epimerizes all ketoses at the C-3 position, and is a unique enzyme having an extremely broad substrate specificity so as to act on D-fructose and D-psicose, L-sorbose and L-tagatose, D-tagatose and D-sorbose, L-psicose and L-fructose, which could not be synthetically connected so far. Because of the finding of this DTE, all the monosaccharides are linked together in a ring, and structuring of the knowledge of monosaccharides is completed, which was named Izumoring.
When taking a close look at Fig. 12, it is found that there are L-forms at the left side, D-forms at the right side and DL-forms in the middle, and further L-forms and D-forms are symmetric with respect to the central point (asterisk) of the ring. For example, D-glucose and L-glucose are symmetric with respect to the central point. Further, the value of Izumoring is that it becomes a plan diagram for production of all the monosaccharides. In the previous example, if L-glucose is intended to be produced from D-glucose as a starting material, it is indicated that D-glucose is isomerized, epimerized, reduced, oxidized, epimerized and isomerized, whereby L-glucose can be produced.
By using Izumoring of monosaccharides having 6 carbon atoms (hexoses), the correlation between sugars that exist in a large amount in nature and rare sugars that exist only in a small amount in nature is shown. D-glucose, D-fructose, D-mannose and D-galactose that can be produced from lactose in milk exist in a large amount in nature, and the other sugars are classified into a rare sugar that exists only in a small amount in nature. Because of the finding of DTE, D-fructose and D-psicose are produced from D-glucose, and further it became possible to produce D-allose, allitol and D-talitol.
When the meanings of Izumoring of monosaccharides having 6 carbon atoms (hexoses) are summarized, they include as follows. Based on the production process and molecular structure (D-form and L-form), all the monosaccharides are put in order structurally (structuring of knowledge), whereby the overall picture of monosaccharides can be understood; an effective and efficient approach for study can be selected; the optimum production pathway can be designed; and a missing portion can be predicted.

D-psicose is a sugar that can be produced on a large scale at present among rare sugars. Psicose is one of the hexoses having a ketone group among monosaccharides. It is known that this psicose exists as optical isomers in D-form and L-form. Here, although D-psicose is a known substance, it rarely exists in nature, therefore, it is defined as a "rare sugar" according to the definition of International Society of Rare Sugars. D-psicose is the D-form of psicose classified into a ketose and is a hexose (C6H12O6). Such D-psicose may be obtained by any means including one extracted from nature, one synthesized by a chemical or biological synthesis method and the like. In a relatively easy way, for example, one prepared by a method using epimerase (e.g. , see JP-A-6-125776) can be employed. The obtained D-psicose liquid can be purified by a method such as deproteinization, decoloration or demineralization as needed, and then the resulting liquid is concentrated, whereby a D-psicose product in a syrup form can be collected. Further, by carrying out fractionation and purification by column chromatography, a product with a high purity of 99% or higher can be easily obtained. Such D-psicose can be used as a monosaccharide as it is.

The present invention relates to a sweetener, a seasoning, a food additive, a food material, a food or drink, a health food or drink, a drug and a feed that can be used for preventing and treating a disease such as arteriosclerosis based on a risk factor such as diabetes, hyperlipemia or hypertension. As for the preventive agent or therapeutic agent, it is used as such, and other than this, it is formulated into a preparation by blending an appropriate additive such as a common excipient, a stabilizer, a preservative, a binder or a disintegrant and selecting an appropriate dosage form such as a liquid, a capsule, a granule, a pill, a powder or a tablet and can be orally or enterally administered. As for the dose, in the case of oral administration, it is preferably 0.3 to 50 g per day per adult human in terms of D-psicose via oral administration, however, it can be appropriately increased or decreased depending on the age and symptoms.
The preparation (drug) of the present invention can efficiently inhibit, for example, the expression of a chemokine or a cytokine and also its toxicity is low. The inhibition of the expression of a chemokine or a cytokine includes inhibition of the expression of mRNA of a chemokine or a cytokine, inhibition of the production of a chemokine or a cytokine, inhibition of the expression of DNA of a chemokine or a cytokine, inhibition of the expression promoter of a chemokine or a cytokine, inhibition of the secretion of a chemokine or a cytokine, inhibition of the translation of mRNA of a chemokine or a cytokine and the like.

The feed of the present invention is a feed for a breeding animal such as livestock, poultry, and other than these, honeybee, silkworm or fish, and is characterized in that the composition blended with D-psicose and/or a mixture thereof is blended such that the content of D-psicose becomes 0.1 to 50% by weight of the amount of the carbohydrates (sugar mass) in a food or drink. In the case where such a feed is administered to a feed animal for a breeding animal such as livestock, poultry, and other than these, honeybee, silkworm or fish, a tendency of obesity will be alleviated. Therefore, the feed of the present invention is a feed useful for preventing obesity or diabetes in pets or obtaining animal meat with less fat.

As for an incorporation method in the form of the composition blended with D-psicose and/or a mixture thereof in a sweetener, a seasoning, a food additive, a food material, a food or drink, a health food or drink, a drug or a feed as described above, it is only necessary to incorporate the composition at 0.1% by weight or more, preferably at 0.5% by weight or more in terms of D-psicose in the process until such a product is completed, and for example, a known method such as mixing, kneading, dissolving, melting, immersing, impregnating, spraying, applying, coating, nebulizing, injecting, crystallizing or solidifying can be appropriately selected.
In the composition blended with D-psicose and/or a mixture thereof of the present invention, D-psicose is blended such that it contained in the composition at 0.1 to 50% by weight, preferably at 0.5 to 30% by weight, more preferably at 1 to 10% by weight. In the composition, when the content of D-psicose is less than 0.1% by weight, an action of inhibiting the expression of a chemokine by a cytokine and inhibiting the secretion of a chemokine (mRNA and a promoter) is not sufficient. Further, in the composition, when the content of D-psicose exceeds 50% by weight, it is not preferred in terms of an economic point of view.

In the case where a rare sugar D-psicose is used as one component of a food, an effective amount of the rare sugar D-psicose can be safely taken in the daily diet. The reason is that the rare sugar D-psicose is a ketohexose, and is a compound with high safety that can be administered to human from this viewpoint.
In the development of drugs foods and the like, the most important and the biggest hurdle is the verification of safety of a rare sugar as a novel substance. A mutagenicity test, a biodegradability test and three types of acute toxicity tests (an oral acute toxicity test, a primary skin irritation test and a primary eye irritation test) are defined as the most basic safety tests. A rare sugar is a monosaccharide that exists in nature even in a small amount, therefore, it can be predicted that it will be safe. However, it is necessary to perform verification precisely. We asked the designated organization to perform the basic safety tests for three types of rare sugars, D-psicose, D-allose and allitol among rare sugars. As a result, it was confirmed that there are not any problems in the safety of any of the rare sugars.

The preparation containing a rare sugar D-psicose or a pharmacologically acceptable salt thereof or/and a hydrate thereof of the present invention (hereinafter abbreviated as the compound of the present invention) will be described in more detail.
As the dosage form of an angiogenesis inhibitor of the present invention, the active ingredient is formulated into any of a variety of forms containing a medically acceptable additive such as a carrier, an excipient, a lubricant or a binder, for example, a liquid in which all the ingredients are dissolved in water or any of a variety of preparations for infusion, a powder, a granule, a tablet, an injection, a suppository, a preparation for external use or the like can be produced by a known drug production technique.

In the case of administering a rare sugar of the present invention by way of injection, an aqueous injection, an aqueous suspension injection, fat emulsion, a liposome injection and the like are preferred. As for the aqueous injection or the aqueous suspension injection, a rare sugar according to the present invention, its derivative or a pharmacologically acceptable salt thereof is mixed with purified water, and according to need, a water-soluble or water-swellable polymer, a pH adjuster, a surfactant, an osmotic regulator, an antiseptic agent, a preservative or the like is added thereto, and dissolved or suspended by mixing, and if necessary, by heating, and the mixture is sterilized, and then, packed and sealed in an injection container, whereby an aqueous injection or an aqueous suspension injection is prepared. The aqueous injection can be administered intravenously, subcutaneously, intramuscularly, intradermally, or into a joint cavity or the like. Further, the aqueous suspension injection can be administered subcutaneously, intramuscularly, intradermally, or into a joint cavity or the like. Further, they can be administered orally.

As the water-soluble or water-swellable polymer, gelatin, a cellulose derivative, an acrylic acid derivative, povidone, macrogol, a polyamino acid derivative or a polysaccharide is preferred. As the gelatin, purified gelatin is preferred. As the cellulose derivative, methylcellulose, hydroxypropyl methylcellulose 2910, hydroxypropyl methylcellulose 2208, hydroxypropyl methylcellulose 2906, hydroxypropyl cellulose, a low-substituted hydroxypropyl cellulose or sodium carmellose; as the acrylic acid derivative, an aminoacryl methacrylate copolymer or methacrylic acid copolymer; and as the polyamino acid derivative, polylysine or polyglutamic acid are preferred. As the polysaccharide, hyaluronic acid, dextran or dextrin is particularly preferred. The amount of the water-soluble or water-swellable polymer to be added varies depending on the property or amount of esculetin, its derivative or a pharmacologically acceptable salt thereof and the property, molecular weight or application part of the water-soluble or water-swellable polymer, however, in general, it can be used in a range from 0.01% to 10% of the total amount of the preparation.

As the pH adjuster, an acid or an alkali which is harmless to the human body is used, and as the surfactant, a nonionic surfactant, an anionic surfactant or an amphoteric surfactant is used. Further, as the osmotic regulator, sodium chloride, glucose or the like, as the antiseptic agent, a paraben, and as the preservative, ascorbic acid or a sulfite can be exemplified. The used amount thereof is not particularly limited, however, they can be used in an amount falling within a range that allows their actions to be exhibited, respectively. Further, according to need, a local anesthetic such as procaine hydrochloride, a soothing agent such as benzyl alcohol, a chelating agent, a buffer, a water-soluble organic solvent or the like may be added.

The fat emulsion is prepared by blending an emulsifier, and a rare sugar or a pharmacologically acceptable salt thereof in an appropriate fat and oil, adding purified water thereto, and according to need, adding an water-soluble or water-swellable polymer, a pH adjuster, a surfactant, an osmotic regulator, an antiseptic agent, a preservative or the like thereto, emulsifying the mixture using an appropriate emulsifying device, sterilizing the emulsified mixture, and then, packing and sealing it in an injection container.

As for an oral preparation comprising the compound of the present invention, it can be produced by selecting as the dosage form containing the compound of the present invention as the base agent, an appropriate preparation such as a tablet, a powder, a granule, a capsule, a solution, a syrup, an elixir or an oil-based or water-based suspension depending on the administration route, and by employing a known drug production technique together with a common additive such as an excipient, a lubricant or a binder.
As for a solid preparation, it contains a pharmaceutically acceptable additive as well as an active compound, and for example, it can be formulated into a preparation by selecting and mixing a filler, an expander, a binder, a disintegrant, a solubilizer, a moisturizing agent or a lubricant as needed.
Further, as the preparation for external use, a solution, a suspension, an emulsion, an ointment, a gel, a cream, a lotion, a spray and the like can be exemplified.

The present invention will be described in detail with reference to Examples. The present invention is by no means limited by these Examples.

### Example 1

An effect of D-psicose on a group of scavenger receptors and an HDL receptor was examined and new study results were obtained with regard to the antiatherogenic action of D-psicose as described below.

### <Method>

Cell culture: A human liver-derived cell line (Hep G2), a human monocyte-derived cell line (THP-1) and a human vascular smooth muscle cell line (ISS10) were cultured in accordance with the previous reports (13-15) under the conditions of a culture medium DMEM + 10% FBS or RPMI1640 + 10% FBS.
HepG2, THP-1 or ISS10 cells were dispensed into culture dishes at a density of about 70% and subjected to the following experiments.
1. ISS10 cells and THP-1 cells were cultured in control (5.6 mM D-glucose), high glucose (22.4 mM D-glucose) and D-psicose (22.4 mM) for 3 days and examined by the Western blot analysis method in the previous report (15) for the expression of a scavenger receptor A (SRA) in ISS10 cells and a scavenger receptor CD36 in THP-1 cells using commercially available antibodies to SRA and CD36.
   In ISS10 cells, a PMA treatment was carried out as a positive control for induction of the expression of SRA. Further, cyclophilin A was used as a control protein.
2. Hep G2 cells were cultured in the presence of various concentrations of D-glucose and D-psicose and the expression of an HDL receptor CLA-1 was examined by the Western blot analysis method in the previous report (15).
As for the antibody for detection, an antibody obtained by integrating amino acid residues of 185 to 300 in the extracellular domain of CLA-1 into a GST-fusion vector and immunizing a guinea pig with the resulting vector was used.
Further, by integrating a 1200 bp promoter region of the CLA-1 gene into a luciferase reporter gene, introducing the gene into a cell and measuring the luciferase activity, the transcriptional activity of CLA-1 was determined.

### <Results>

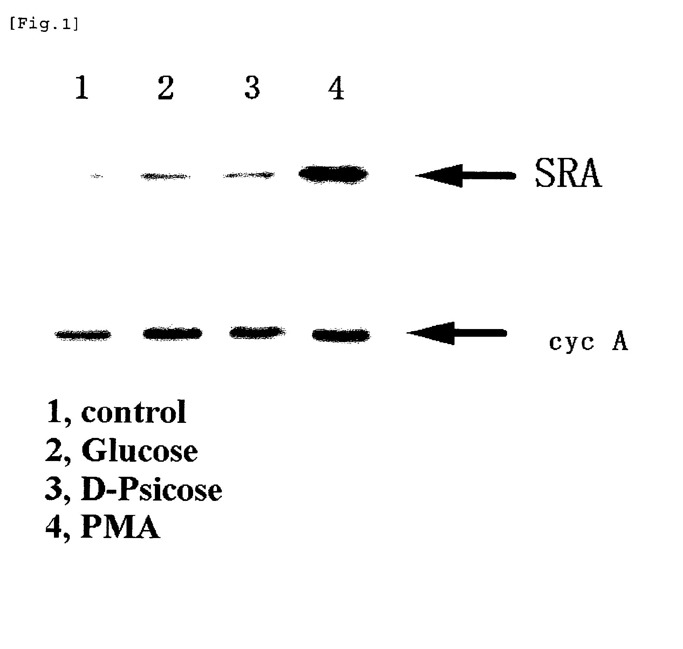

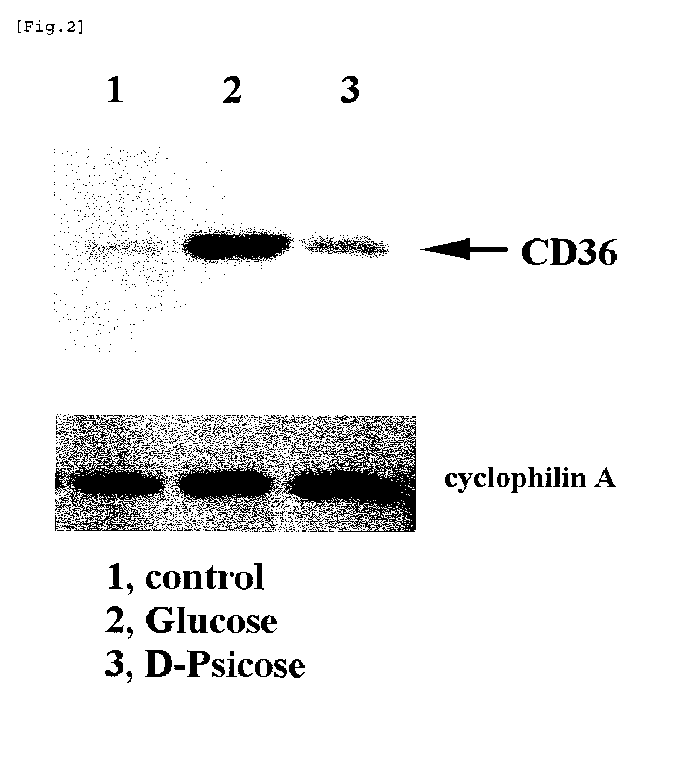

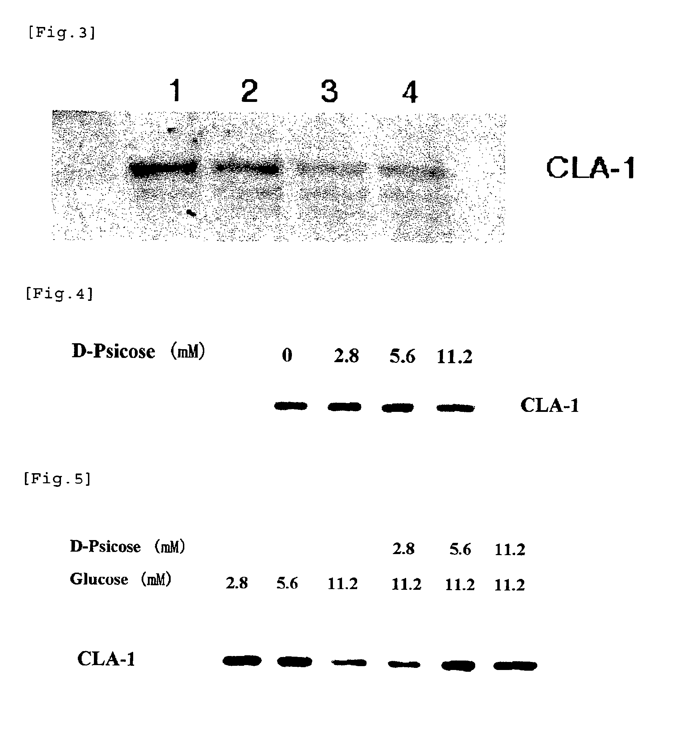

1. The vascular smooth muscle cell line ISS10 was treated with 5.6 mM D-glucose (control), 22.4 mM D-glucose (glucose), 22.4 mM D-psicose (D-psicose) or 100 nM PMA (PMA) for 72 hours and the expression of SRA was examined by the Western blot analysis method.
   As shown in Fig. 1, the expression of SRA was induced by PMA, however, no effect was observed by D-glucose and D-psicose.
2. On the other hand, as for CD36, the examination was carried out using the monocyte-derived cell line THP-1.
   In the same manner as the previous reports, the expression of CD36 was induced by a high concentration of D-glucose, however, the expression of CD36 was not enhanced by D-psicose (Fig. 2).
3. The human hepatocyte-derived cell line HepG2 was cultured with 2.8 mM (lane 1), 5.6 mM (lane 2), 11.2 mM (lane 3), or 22 . 4 mM (lane 4) D-glucose for 72 hours and the expression of CLA-1 was examined by the Western blot analysis method. As shown in Fig. 3, the expression of CLA-1 was reduced by 11.2 mM (lane 3) and 22.4 mM (lane 4) D-glucose.
4. Then, HepG2 was cultured with 0, 2.8, 5.6 or 11.2 mM D-psicose for 72 hours and the expression of CLA-1 was examined by the Western blot analysis method. As shown in Fig. 4, a reduction in CLA-1 was not observed by D-psicose.
5. HepG2 was cultured with 2.8, 5.6, or 11.2 mM D-glucose or 11.2 mM D-glucose supplemented with 2.8, 5.6 or 11.2 mM D-psicose for 72 hours and the expression of CLA-1 was examined by the Western blot analysis method. As shown in Fig. 5, CLA-1 was reduced by 11.2 mM D-glucose, however, by adding D-psicose, the inhibition of CLA-1 was released in a concentration dependent manner.
6. The transcriptional activity of CLA-1 was also examined. The luciferase reporter gene containing the CLA-1 promoter was introduced into HepG2 cells and the resulting HepG2 cells were cultured with 2.8 mM (lane 1), 5.6 mM (lane 2), 11.2 mM (lane 3), 22.4 mM (lane 4) D-glucose or 22.4 mM D-psicose for 24 hours and the luciferase activity was measured. A high concentration of D-glucose inhibited the activity of the CLA-1 promoter. On the other hand, D-psicose did not affect the activity of the CLA-1 promoter (Fig. 6).

### <Discussion>

The trigger of the onset of arteriosclerosis is the migration of monocytes to the vascular wall, cholesterol accumulation by scavenger receptors and foam cell formation of macrophages (Fig. 7). To date, as oxidized LDL receptors (scavenger receptors), SRA, CD36, CD68, LOX, SRC, SR-B1 and the like have been cloned (Fig. 9). In this study, an effect of D-psicose on the expression of scavenger receptors was examined. As for SRA, the vascular smooth muscle cells were cultured with D-psicose or D-glucose and the expression of SRA was examined by the Western blot analysis method, however no apparent difference was observed.
As for CD36, the examination was carried out using the monocyte-derived cell line THP-1. According to the reports, the expression thereof was induced by a high concentration of glucose, however D-psicose did not enhance the expression of CD36 (Fig.2). As a correlation between diabetes and arteriosclerosis, induction of the expression of a scavenger receptor by high blood sugar levels has been pointed out, however, it is considered that D-psicose does not have an action of inducing a scavenger receptor. Clinically, with regard to the diet therapy for patients with diabetes, it is considered that if D-psicose can be used as an alternative sugar to glucose, it is useful for the prevention of complications.

In the living body, as an antiatherogenic action, the reverse cholesterol transport system mediated by HDL is known (Fig. 10). The human gene CLA-1, which we identified, mediates cholesterol uptake in the liver as an HDL receptor. Therefore, the human liver-derived cell line HepG2 cells were treated with various concentrations of glucose or D-psicose and the expression of CLA-1 was examined. A high concentration of glucose inhibited the expression of CLA-1, however, D-psicose released the inhibition of the expression of CLA-1 by glucose. Clinically, in patients with diabetes, it has been pointed out that the reverse cholesterol transport system is impaired. At present, no agent that activates the reverse cholesterol transport system in vivo is known. It was considered that the mechanism of the inhibition of the reduction in CLA-1 by D-psicose is useful for therapy aiming at the inhibition of the onset of arteriosclerosis in patients with diabetes.

### Brief Description of the Drawings

[Fig. 1] Fig. 1 is a view showing that the expression of SRA was induced by PMA, but no effect was observed by D-glucose and D-psicose.
[Fig. 2] Fig. 2 is a view showing that the expression of CD36 was induced by a high concentration of glucose, but the expression of CD36 was not enhanced by D-psicose.
[Fig. 3] Fig. 3 is a view showing that the expression of CLA-1 was reduced by 11.2 mM (lane 3) and 22.4 mM (lane 4) D-glucose. [Fig. 4] Fig. 4 is a view showing that a reduction in CLA-1 was not observed by D-psicose.
[Fig. 5] Fig. 5 is a view showing that CLA-1 was reduced by 11.2 mM D-glucose, but by adding D-psicose, inhibition of CLA-1 was released in a concentration dependent manner.
[Fig. 6] Fig. 6 is a view showing that a high concentration of D-glucose inhibited the activity of the CLA-1 promoter, but D-psicose did not affect the activity of the CLA-1 promoter.
[Fig. 7] Fig. 7 is a view showing that the trigger of the onset of arteriosclerosis is the migration of monocytes to the vascular wall and cholesterol accumulation by scavenger receptors and foam cell formation of macrophages.
[Fig. 8] Fig. 8 is a view showing that as the stimulation of MCP-1 secretion, inflammatory cytokines, TNF-α and IL-1β are known.
[Fig. 9] Fig. 9 is a view showing that a scavenger receptor, which is a receptor for oxidized LDL that is an arteriosclerosis inducer, is induced to be expressed by macrophages in the arteriosclerotic lesion and plays the leading role in the formation of arteriosclerotic lesion.
[Fig. 10] Fig. 10 is a view showing that as an antiatherogenic action in vivo, the reverse cholesterol transport system mediated by high density lipoprotein (HDL) is known.
[Fig. 11] Fig. 11 is a linkage diagram of Izumoring.
[Fig. 12] Fig. 12 is a diagram for illustrating Izumoring of C6 at the lower portion of Fig. 11.

## Claims

1. A composition comprising D-psicose, which is a rare sugar as an active ingredient for an antiatherogenic action, for use in treating arteriosclerosis in patients with diabetes.

2. The composition for use in treating arteriosclerosis according to claim 1, wherein the antiatherogenic action includes an action of inhibiting or reducing the expression of a chemokine or a cytokine related to arteriosclerosis *in vivo* and an action of promoting the expression of a receptor involved in the improvement of arteriosclerosis without affecting the expression of an arteriosclerosis inducer.

3. The composition for use in treating arteriosclerosis according to claim 2, wherein the action of promoting the expression is an action of inhibiting the induction of the expression of a chemokine by a cytokine related to arteriosclerosis *in vivo* and promoting the expression of a receptor involved in the improvement of arteriosclerosis without affecting the expression of a scavenger receptor CD36 which is an arteriosclerosis inducer by D-psicose.

4. The composition for use in treating arteriosclerosis according to claim 1, 2 or 3, wherein D-psicose is used in the form of a composition blended with D-psicose.

5. The composition for use in treating arteriosclerosis according to claim 4, wherein the composition is in the form of a product selected from the group consisting of sweeteners, seasonings, food additives, food materials, foods and drinks, health foods and drinks, drugs, and feeds blended with D-psicose as the active ingredient.

## Patentansprüche

1. Zusammensetzung, die D-Psicose, welche ein seltener Zucker ist, als ein Wirkstoff für eine antiatherogene Wirkung umfasst, für die Verwendung bei der Behandlung von Arteriosklerose bei Patienten mit Diabetes.

2. Zusammensetzung für die Verwendung in der Behandlung von Arteriosklerose gemäß Anspruch 1, wobei die antiatherogene Wirkung eine Wirkung der Hemmung oder Verringerung der Expression eines Chemokins oder eines Cytokins mit Bezug auf Arteriosklerose *in vivo* und eine Wirkung der Förderung der Expression eines Rezeptors, der an der Verbesserung der Arteriosklerose ohne Beeinträchtigung der Expression eines Arterioskleroseinduktors beteiligt ist, beinhaltet.

3. Zusammensetzung für die Verwendung in der Behandlung von Arteriosklerose nach Anspruch 2, wobei die Wirkung der Förderung der Expression eine Wirkung der Hemmung der Induktion der Expression eines Chemokins durch ein Cytokin mit Bezug auf Arteriosklerose *in vivo* und die Förderung der Expression eines Rezeptors, der an der Verbesserung einer Arteriosklerose ohne Beeinträchtigung der Expression eines Scavenger-Rezeptors CD36 beteiligt ist, welcher ein Arterioskleroseinduktor ist, durch D-Psicose ist.

4. Zusammensetzung für die Verwendung in der Behandlung von Arteriosklerose nach Anspruch 1, 2 oder 3, wobei D-Psicose in der Form einer mit D-Psicose gemischten Zusammensetzung verwendet wird.

5. Zusammensetzung für die Verwendung in der Behandlung von Arteriosklerose nach Anspruch 4, wobei die Zusammensetzung in der Form eines Produkts ausgewählt aus der Gruppe bestehend aus Süßstoffen, Gewürzen, Lebensmittelzusätzen, Lebensmittelmaterialien, Lebensmitteln und Getränken, Gesundheitslebensmitteln und -getränken, Arzneimitteln und Futterstoffen gemischt mit D-Psicose als der Wirkstoff ist.

## Revendications

1. Composition comprenant de la D-psicose, qui est un sucre rare en tant qu'ingrédient actif pour une action antiathérogène, destinée à être utilisée dans le traitement de l'artériosclérose chez des patients diabétiques.

2. Composition destinée à être utilisée dans le traitement de l'artériosclérose selon la revendication 1, dans laquelle l'action antiathérogène comporte une action d'inhibition ou de réduction de l'expression d'une chémokine ou d'une cytokine relative à l'artériosclérose in vivo et une action de favorisation de l'expression d'un récepteur impliqué dans l'amélioration de l'artériosclérose sans affecter l'expression d'un inducteur d'artériosclérose.

3. Composition destinée à être utilisée dans le traitement de l'artériosclérose selon la revendication 2, dans laquelle l'action de favorisation de l'expression est une action d'inhibition de l'induction de l'expression d'une chémokine par une cytokine relative à l'artériosclérose in vivo et de favorisation de l'expression d'un récepteur impliqué dans l'amélioration de l'artériosclérose sans affecter l'expression d'un récepteur épurateur CD36 qui est un inducteur d'artériosclérose par D-psicose.

4. Composition destinée à être utilisée dans le traitement de l'artériosclérose selon la revendication 1, 2 ou 3, dans laquelle la D-psicose est utilisée sous la forme d'une composition mélangée avec de la D-psicose.

5. Composition destinée à être utilisée dans le traitement de l'artériosclérose selon la revendication 4, dans laquelle la composition est sous la forme d'un produit choisi dans le groupe constitué d'édulcorants, d'assaisonnements, d'additifs alimentaires, de matières alimentaires, d'aliments et de boissons, d'aliments et de boissons diététiques, de médicaments, et d'aliments mélangés avec de la D-psicose, en tant qu'ingrédient actif.
